# EUROPÄISCHE PATENTSCHRIFT

(11) **EP 3 470 105 B1**
(45) Veröffentlichungstag und Bekanntmachung des Hinweises auf die Patenterteilung: **22.04.2020**
(21) Anmeldenummer: 17196410.9
(22) Anmeldetag: 13.10.2017
(51) Int. Cl.: A61M 25/00, A61F 2/966, A61M 25/06, A61F 2/24

(54) **EINFÜHRELEMENT FÜR EINE MEDIZINISCHE EINFÜHREINRICHTUNG**
INSERTION ELEMENT FOR A MEDICAL INSERTION DEVICE
ÉLÉMENT D'INTRODUCTION POUR UN DISPOSITIF D'INTRODUCTION MÉDICALE

(43) Veröffentlichungstag der Anmeldung: 17.04.2019
(73) Patentinhaber: BIOTRONIK SE & Co. KG, 12359 Berlin (DE)
(72) Erfinder: Keller, Mark, 5000 Aarau (CH)
(74) Vertreter: Randoll, Sören

(56) Entgegenhaltungen:
- EP-A2- 2 676 641
- US-A1- 2013 338 677
- US-A1- 2016 121 080
- US-B1- 8 012 143

## Beschreibung

Die Erfindung betrifft ein Einführelement gemäß Anspruch 1, eine Einführeinrichtung gemäß Anspruch 11 sowie ein Verfahren gemäß Anspruch 12.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Bei diesen Implantaten handelt es sich z. B. um Herzimplantate, Gefäßprothesen, Stents oder sonstige geeignete Implantate, die vor dem Einführen in den Körper mit einer Einführeinrichtung, insbesondere einem Katheter, verbunden werden und mit deren bzw. dessen Hilfe am Einsatzort genau platziert und definiert freigegeben werden.

Zum Einschleusen in den tierischen und/oder menschlichen Körper wird hierzu z. B. ein schlauchartiges Einführelement eingesetzt, durch welches das Implantat mittels der Einführvorrichtung geschoben wird.

Das Einführelement bzw. die Einführeinrichtung fährt dabei während des sogenannten Trackings, also beim Einschieben des Einführelementes in ein Körperlumen, eine (anatomisch bedingt) im Raum verschieden gekrümmte Bahn ab. Um diese Kurvenbahn mit möglichst wenig Reibungswiderstand passieren zu können, werden u. a. die Systeme auf ihrer Länge, d. h. in der axialen Richtung, in der Biegesteifigkeit variiert. Dies wird in der Regel über die Wahl unterschiedlicher Kunststoffe mit verschiedenen Flexural Moduli (Elastizitätsmodule) bewerkstelligt.

Hierbei erweist es sich allerdings als problematisch, dass dabei in einer Übergangszone (Materialübergang bzw. Fügestelle) ein Steifigkeitssprung im Einführelement bzw. Katheterschlauch hervorgerufen wird, welcher für den Schadensmechanismus Beulen und Knicken eine Schwachstelle darstellt.

Um die Steifigkeit eines Einführelements bzw. Katheters variieren zu können, werden nebst unterschiedlich biegesteifen Kunststoffen auch geflochtene Schläuche beziehungsweise Geflechte in die Katheterwandung als Armierung eingelassen, welche wiederum in der Steigung (Flechtwinkel) variiert werden können. Das Material und die Dimension des Flechtdrahtes bleiben dabei konstant bzw. können nur mit technisch sehr aufwändigen Methoden/Verfahren graduell variiert werden.

EP 2676641 beispielsweise zeigt ein Einführelement, welches zum Zusammenwirken mit einer Einführvorrichtung zum Einführen eines medizinischen Implantats in einen menschlichen oder tierischen Körper ausgebildet ist. Das Einführelement umfasst eine Hülle, welche in Umfangsrichtung wenigstens zwei Arten von Umfangssegmenten mit unterschiedlicher Elastizität aufweist mit unterschiedlicher Elastizität aufweist

Hiervon ausgehend liegt der vorliegenden Erfindung daher die Aufgabe zugrunde, ein Einführelement bzw. ein Verfahren zur Herstellung eines solchen Elementes bereitzustellen, bei dem die vorgenannten Nachteile gemindert sind.

Diese Aufgabe wird durch ein Einführelement mit den Merkmalen des Anspruchs 1, eine Einführeinrichtung mit den Merkmalen des Anspruchs 11 sowie durch ein Verfahren mit den Merkmalen des Anspruchs 12 gelöst. Vorteilhafte Ausgestaltungen der Erfindung sind in den entsprechenden Unteransprüchen angegeben und werden nachfolgend beschrieben.

Es wird ein Einführelement für eine medizinische Einführeinrichtung vorgeschlagen, die zum Positionieren eines medizinischen Implantats an einem Zielort in einem menschlichen oder tierischen Körper ausgebildet ist, wobei das Einführelement eine in einer axialen Richtung erstreckte Hülle aufweist, die ein in der axialen Richtung erstrecktes Lumen bzw. Innenraum der Hülle umgibt, wobei die Hülle zumindest einen vorzugsweise schlauchförmigen ersten Abschnitt und einen den ersten Abschnitt in der axialen Richtung gegenüberliegenden (vorzugsweise schlauchförmigen) zweiten Abschnitt aufweist, wobei die beiden Abschnitte über einen in der Umfangsrichtung der Hülle umlaufenden Verbindungsbereich miteinander verbunden sind, in dem die beiden Abschnitte ineinander übergehen, und wobei der erste Abschnitt aus einem ersten Material und der zweite Abschnitt aus einem zweiten Material gefertigt ist, wobei das erste Material eine größere Biegesteifigkeit aufweist als das zweite Material. Erfindungsgemäß ist hierbei vorgesehen, dass der Verbindungsbereich mäanderförmig in der Umfangsrichtung umläuft.

Hierbei ist bevorzugt vorgesehen, dass der mäanderförmige Verlauf des Verbindungsbereiches durch alternierend in der Umfangsrichtung der Hülle hintereinander angeordnete berg- und talförmige Abschnitte des Verbindungsbereiches gebildet ist. Es ist ferner bevorzugt vorgesehen, dass die beiden Abschnitte im Verbindungsbereich mit einander verschweißt sind.

In einer bevorzugten Ausführungsform ist das Einführelement aus zwei Bereichen aufgebaut, die über einen Verbindungbereich miteinander verbunden sind. In weiteren Ausführungsformen können auch mehr als zwei Bereich miteinander so verbunden, dass zwei Außenbereich vorgesehen sind und über mindestens einen Innenbereich mit einander verbunden sind. Bei drei Bereichen liegen somit die zwei Außenbereiche vor, die über einen Innenbereich wie hierin beschrieben verbunden sind. Bei vier Bereichen liegen demnach die zwei Außenbereiche vor, die über zwei Innenbereiche wie hierin beschrieben verbunden sind. Es ist bevorzugt, dass alle Bereiche einen unterschiedlichen, bevorzugt in einer Richtung sich konvergierend verändernden Flexural Modulus aufweisen, also von abstufend von wenig flexibel hin zu flexibel. Es sind jedoch auch andere Ausführungsformen denkbar wie hierin an anderer Stelle näher beschrieben.

Durch die Erfindung wird mit Vorteil bewirkt, dass der Übergangsbereich bzw. der Verbindungsbereich zwischen den mindestens beiden Abschnitten bzw. Materialien eine nicht verschwindende Komponente in der axialen Richtung der Hülle aufweist. Es ergibt sich somit hinsichtlich der Härte oder Biegesteifigkeit der beiden Abschnitte ein gradueller Übergang in axialer Richtung, der sich längenmäßig (bezogen auf die axiale Richtung) vom tiefsten Tal bis zum höchsten Berg des mäanderförmigen Verbindungsbereiches erstreckt, d. h. über die Ausdehnung des mäanderförmigen Verlaufs entlang der axialen Richtung lässt sich die Länge des graduellen Übergangs mit Vorteil einstellen. Weiterhin lässt sich mit Vorteil aufgrund des mäanderförmigen Verlaufs auch eine gewisse Zylindersymmetrie (bezogen auf die axiale Richtung) des Biegesteifigkeitsübergangs erzielen, z. B. indem eine ausreichende Mehrzahl an berg- und talförmigen Abschnitten des mäanderförmigen Verlaufs bzw. Verbindungsbereiches vorgesehen wird.

Gemäß einer bevorzugten Ausführungsform ist vorgesehen, dass eine Basis des jeweiligen bergförmigen Abschnittes kleiner ist als dessen Höhe. Die Basis der bergförmigen Abschnitte liegt dabei auf halber Höhe zwischen dem höchsten Punkt eines bergförmigen Abschnitts und dem tiefsten Punkt eines benachbarten talförmigen Abschnitts.

Weiterhin ist gemäß einer bevorzugten Ausführungsform der Erfindung vorgesehen, dass jeder bergförmige Abschnitt durch je zwei Schenkel gebildet ist, wobei die beiden Schenkel jeweils von der Basis ausgehend aufeinander zu laufen und einen Winkel einschließen, der vorzugsweise im Bereich von 1° bis 60° liegt und weiter bevorzugt im Bereich von 2 bis 45° und am meisten bevorzugt im Bereich von 5 bis 30° liegt. Weiterhin können auch die bergförmigen Abschnitte durch je zwei Schenkel gebildet sein, wobei die beiden Schenkel des jeweiligen talförmigen Abschnitts einen Winkel einschließen, der vorzugsweise im Bereich von 1° bis 60° liegt und weiter bevorzugt im bereich von 2 bis 45° und am meisten bevorzugt im Bereich von 5 bis 30° liegt.

Die einzelnen tal- und bergförmigen Abschnitte des Verbindungsbereiches können jeweils rechteckförmig, trapezförmig, dreieckförmig, oder stetig gekrümmt ausgebildet sein, wobei ein stetig gekrümmter, insbesondere wellenförmiger Verlauf des Verbindungsbereiches bevorzugt ist.

Gemäß einer Ausführungsform des erfindungsgemäßen Einführelementes ist vorgesehen, dass das erste Material ein Kunststoff ist oder einen Kunststoff aufweist, wobei insbesondere der besagte Kunststoff einer der folgenden Kunststoffe ist: ein thermoplastisches Elastomer, Polyetherblockamid (PEBA), ein thermoplastisches Polyurethan, ein Polycarbonat-basiertes thermoplastisches Polyurethan, oder ein Polyether-basiertes thermoplastisches Polyurethan. Bei PEBA handelt es sich um ein thermoplastisches Elastomer, nämlich ein Blockcopolymer, auf Basis eines Polyamids (z. B. PA6, PA11, PA12).

Weiterhin ist gemäß einer Ausführungsform des erfindungsgemäßen Einführelementes vorgesehen, dass das zweite Material ein Kunststoff ist oder einen Kunststoff aufweist, wobei insbesondere der besagte Kunststoff einer der folgenden Kunststoffe ein thermoplastisches Elastomer, Polyetherblockamid (PEBA), ein thermoplastisches Polyurethan, ein Polycarbonat-basiertes thermoplastisches Polyurethan, oder ein Polyether-basiertes thermoplastisches Polyurethan ist.

Weiterhin ist gemäß einer Ausführungsform des erfindungsgemäßen Einführelementes vorgesehen, dass das erste Material einen Flexural Modulus aufweist, der im Bereich von 7 MPa bis 650 MPa liegt.

Weiterhin ist gemäß einer Ausführungsform des erfindungsgemäßen Einführelementes vorgesehen, dass das zweite Material einen anderen Flexural Modulus als das erste Material aufweist, der im Bereich von 7 MPa bis 650 MPa liegt.

In einer Ausführungsform liegt ein Unterschied zwischen dem Flexural Modulus des ersten Materials und dem des zweiten Materials von mindestens 5 MPa vor. In einer weiteren Ausführungsform liegt der Unterschied zwischen dem Flexural Modulus des ersten Materials und dem des zweiten Materials im Bereich von 5 bis 350 MPa, weiter im Bereich von 20 bis 250 MPa und bevorzugt im Bereich von 25 MPa bis 180 MPa.

Weiterhin ist gemäß einer Ausführungsform des erfindungsgemäßen Einführelementes vorgesehen, dass der erste Abschnitt und der zweite Abschnitt über bzw. im Verbindungsbereich materialschlüssig miteinander verbunden sind, insbesondere durch Verschmelzen der beiden Materialien. Es ist demgemäß bevorzugt, dass die Materialien des ersten Abschnittes und des zweiten Abschnittes so ausgewählt sind, dass die Materialien miteinander verschweißbar sind. Eine Verschweißung des ersten und des zweiten Abschnittes hat den Vorteil, dass zum einen eine starke schlüssige Verbindung zwischen den Bereichen eingeführt wird, welche zum zweiten verfahrensseitig automatisch und dadurch ökonomisch vorteilhaft hergestellt werden kann.

Der Verbindungsbereich kann auch als Übergangsbereich bezeichnet werden, da hier (z. B. verschmelzungsbedingt) ein Wechsel vom ersten zum zweiten Material stattfindet.

Gemäß einem weiteren Aspekt der vorliegenden Erfindung ist eine Einführeinrichtung vorgesehen, die ein erfindungsgemäßes bzw. hierin beschriebenes Einführelement aufweist, und dazu ausgebildet ist, ein medizinisches Implantat an einem Zielort in einem menschlichen oder tierischen Körper zu Positionieren und insbesondere Freizusetzen, wobei das Einführelement zumindest eine der folgenden Komponenten der Einführeinrichtung bildet oder zumindest einen Teil einer der folgenden Komponenten der Einführeinrichtung bildet:
- ein Innenschaft zum Tragen des Implantats, wobei insbesondere der Innenschaft in ein Außenschaft der Einführeinrichtung geführt ist;
- ein Außenschaft, der einen Innenschaft der Einführeinrichtung umgibt, der zum Tragen des Implantats ausgebildet ist;
- eine Schleuse, über die ein Außenschaft der Einführeinrichtung zusammen mit einem im Außenschaft geführten Innenschaft der Einführeinrichtung, der zum Tragen des Implantats dient, in ein Körperlumen eines menschlichen oder tierischen Körpers einführbar ist;
- ein Stabilisator, der zum Stabilisieren eines Außenschafts der Einführeinrichtung dient und den Außenschaft bevorzugt umgibt, wobei im Außenschaft ein Innenschaft der Einführeinrichtung geführt ist, der zum Tragen des Implantats dient.

Vorzugsweise ist vorgesehen, dass der jeweils weniger harte zweite Abschnitt jeweils einen distalen Abschnitt der jeweiligen Komponente bildet, d. h. einen Abschnitt, der entlang der Einführeinrichtung weiter weg von einem Bediener bzw. einer Handhabeeinrichtung der Einführeinrichtung angeordnet ist, als ein proximaler Abschnitt der Komponente. Die Einführeinrichtung ist insbesondere dazu konfiguriert, mittels der Handhabeeinrichtung (durch den Bediener) bedient zu werden, wobei insbesondere die Handhabeeinrichtung hierfür geeignete Betätigungsmittel aufweist.

Gemäß einem weiteren Aspekt der vorliegenden Erfindung wird ein Verfahren zur Herstellung eines erfindungsgemäßen Einführelementes offenbart, das die Schritte aufweist:
- Bereitstellen eines ersten und eines zweiten Abschnittes einer Hülle eines herzustellenden Einführelementes, wobei die Abschnitte vorzugsweise schlauchförmig ausgebildet sind, wobei der erste Abschnitt aus einem ersten Material und der zweite Abschnitt aus einem zweiten Material gebildet ist, wobei das erste Material eine größere Biegesteifigkeit als das zweite Material aufweist, wobei der erste Abschnitt eine umlaufende Stirnseite mit einem mäanderförmigen Verlauf ausweist, und wobei der zweite Abschnitt eine Stirnseite mit einem mäanderförmigen Verlauf aufweist, der komplementär zum Verlauf der ersten Stirnseite ausgebildet ist, so dass die beiden Stirnseiten formschlüssig aneinander anliegen können,
- Anordnen der beiden Abschnitte zueinander, derart, dass die beiden Stirnseiten formschlüssig aneinander anliegen, und
- Ausbilden einer (vorzugsweise materialschlüssigen) Verbindung zwischen den beiden Stirnseiten, so dass die beiden Abschnitte zu einer einstückigen Hülle des Einführelementes miteinander verbunden werden, die insbesondere ein durchgängiges Lumen umgibt, das sich vom erste Abschnitt in den zweiten Abschnitt der Hülle erstreckt.

Gemäß einer Ausführungsform des erfindungsgemäßen Verfahrens ist vorgesehen, dass die besagte (insbesondere materialschlüssige) Verbindung durch Verschmelzen der beiden Stirnseiten miteinander hergestellt wird.

Durch das (insbesondere materialschlüssige) Verbinden wird also der besagte mäanderförmige Verbindungs- bzw. Übergangsbereich der Hülle geschaffen, der insbesondere die oben genannten Merkmale bzw. Eigenschaften aufweist.

Bevorzugt wird der mäanderförmige Verlauf der Stirnseiten, der der jeweiligen Stirnseite bzw. dem jeweiligen Abschnitt insbesondere eine kronenartige Geometrie verleiht, mittels eines Lasers in die noch nicht in ihre Endform gebrachten Enden des jeweiligen Abschnittes geschnitten.

Die Abschnitte werden anschließend bevorzugt auf einen ersten Dorn aufgezogen, wobei die Stirnseiten mittels Wärmezufuhr aneinander vorfixiert werden. Der erste Dorn kann im Durchmesser variierbar sein (z. B. konisch). Als Dorn kommen alle geeigneten (insbesondere im Durchmesser variierbaren) Körper in Frage, z. B. auch Ballons etc.. Anschließend wird bevorzugt die aus den beiden Abschnitten gebildete Hülle auf einem zweiten Dorn aufgezogen, der den Enddurchmesser der Hülle aufweist bzw. vorgibt, wobei bevorzugt die Hülle thermisch mittels eines Schrumpfschlauchs auf eine oder mehrere unter der Hülle liegende Schichten aufgebracht wird, z. B. auf einen PTFE Liner, ein laserstrukturiertes Metall- oder Kunststoffrohr, und/oder ein Geflecht. Der zweite Dorn kann ebenfalls im Durchmesser variierbar sein (z. B. konisch). Insbesondere wird der Schrumpfschlauch anschließend wieder entfernt.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine schematische Ansicht eines erfindungsgemäßen Einführelements;
- Fig. 2A - 2C: schematische Darstellungen möglicher weiterer mäanderförmiger Verläufe des Verbindungsbereiches; und
- Fig. 3: eine Ansicht einer Einführeinrichtung, bei der das erfindungsgemäße Einführelement verwendet werden kann;

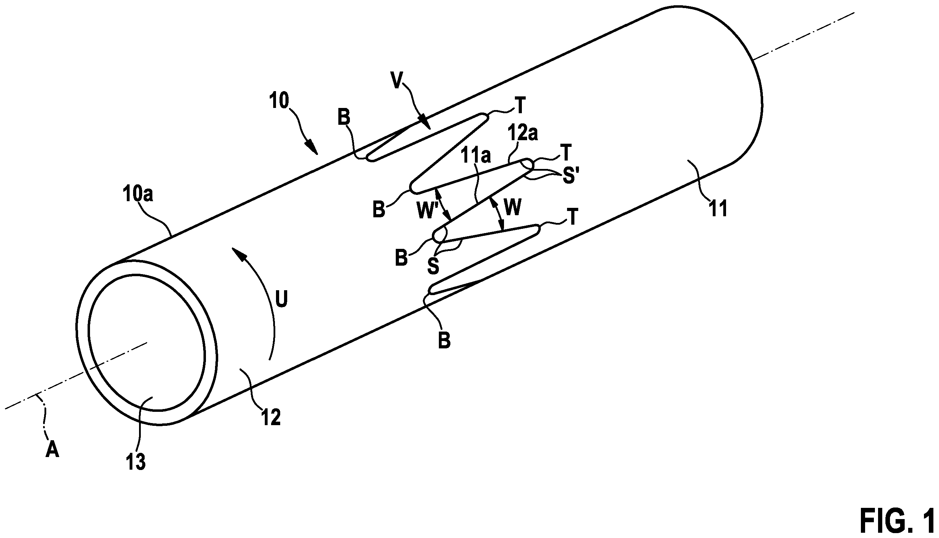

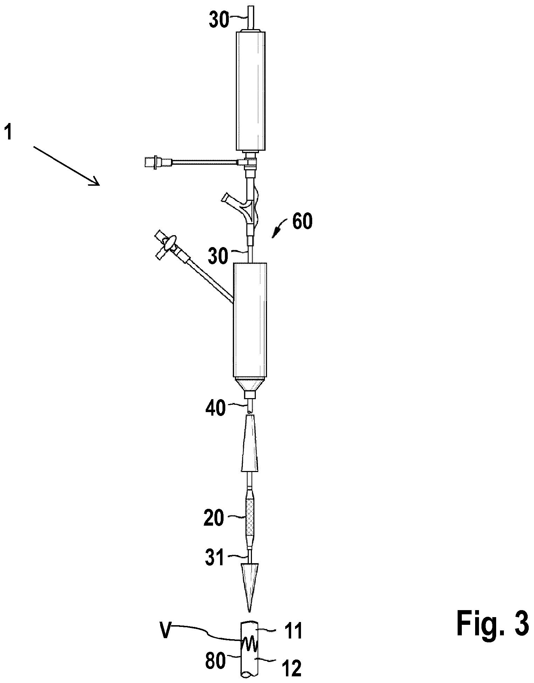

Die Figur 1 zeigt eine Ausführungsform eines erfindungsgemäßen Einführelements 10, das z. B. gemäß Figur 3 in einer Einführeinrichtung 1 verwendet werden kann, die zum Positionieren eines medizinischen Implantats 20 an einem Zielort in einem menschlichen oder tierischen Körper ausgebildet ist.

Wie in der Figur 1 dargestellt ist, weist das Einführelement 10 eine in einer axialen Richtung A erstreckte, z. B. hohlzylindrische oder hohlkonisch, Hülle 10a auf, wobei die Hülle 10a ein durchgängiges Lumen 13 umgibt und einen ersten Abschnitt 11 und einen dem ersten Abschnitt 11 in der axialen Richtung A gegenüberliegenden zweiten Abschnitt 12 aufweist. Hierbei sind die beiden Abschnitte 11, 12 über einen in der Umfangsrichtung U der Hülle 10a umlaufenden Verbindungsbereich V miteinander verbunden, wobei in dem Verbindungsbereich (in axialer Richtung A) der erste Abschnitt 11 in den zweiten Abschnitt 12 übergeht. Weiterhin ist der erste schlauchförmige Abschnitt 11 aus einem ersten Material und der zweite schlauchförmige Abschnitt 12 aus einem zweiten Material gefertigt, wobei das erste Material einen größeren Flexural Modulus aufweist als das zweite Material. Erfindungsgemäß ist nun vorgesehen, dass der besagte Verbindungsbereich V mäanderförmig in der Umfangsrichtung U umläuft. Hierbei weist der mäanderförmig verlaufende Verbindungs- bzw. Übergangsbereich V in der Umfangsrichtung U alternierend angeordnete berg- und talförmige Abschnitte B, T auf.

Gemäß Figur 1 können diese Abschnitte des Verbindungsbereiches V jeweils stetig gekrümmt sein, so dass sich insbesondere ein wellenförmig verlaufender Verbindungsbereich V ergibt, wie er in der Figur 1 gezeigt ist.

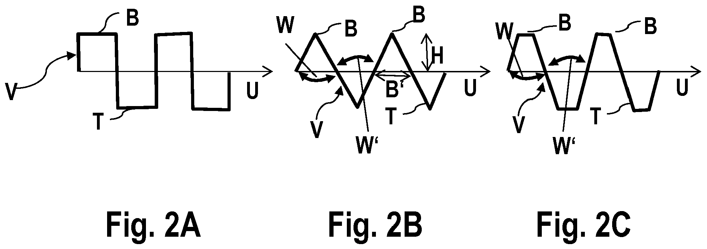

Es sind allerdings auch andere mäanderförmige Verläufe bzw. Geometrien denkbar, wie sie z. B. in den Figuren 2A, 2B und 2C angedeutet sind. Danach kann der Verbindungsbereich V auch einen mäanderförmigen Verlauf mit rechteckförmigen Berg- bzw. Talbabschnitten B, T (Fig. 2A), dreieckförmigen Berg- bzw. Talbabschnitten B, T (Fig. 2B) oder trapezförmigen Berg- bzw. Talbabschnitten B, T (Fig. 2C) aufweisen. Andere Modifikationen dieser prinzipiellen Verläufe sind ebenfalls denkbar.

Bevorzugt ist vorgesehen, wie in den Figuren 1, 2B und 2C angedeutet, dass jeder bergförmige Abschnitt B durch je zwei Schenkel S gebildet ist, wobei die beiden Schenkel S des jeweiligen bergförmigen Abschnitts B einen Winkel W bilden bzw. einschließen können, der vorzugsweise im Bereich von 1° bis 60° liegt und weiter bevorzugt im Bereich von 2 bis 45° und am meisten bevorzugt im Bereich von 5 bis 30° liegt. In gleicher Weise kann jeder bergförmige Abschnitt B durch je zwei Schenkel S' gebildet sein, wobei die beiden Schenkel S' des jeweiligen talförmigen Abschnitts T einen Winkel W' bilden, der vorzugsweise ebenfalls im Bereich von 1° bis 60° liegt und weiter bevorzugt im Bereich von 2 bis 45° und am meisten bevorzugt im Bereich von 5 bis 30° liegt.

Weiterhin kann grundsätzlich vorgesehen sein, wie in der Figur 2B exemplarisch angedeutet, dass die jeweilige Basis B' eines bergförmigen Abschnittes B kleiner ist als dessen Höhe H. Die Basis B' entspricht dabei dem Fuß des jeweiligen bergförmigen Abschnittes B und weist eine Breite auf, die dem Abstand der Schenkel S des jeweiligen bergförmigen Abschnitts B entspricht, und zwar auf halber Höhe zwischen dem höchsten Punkt des bergförmigen Abschnitts B und dem tiefsten Punkt der benachbarten talförmigen Abschnitte T. Die Höhe H ist dabei der Abstand von der Basis B zum höchsten Punkt des bergförmigen Abschnitts B. Die Höhe verläuft also senkrecht zur Umfangsrichtung U bzw. parallel zur axialen Richtung A.

Bevorzugt ist weiterhin vorgesehen, dass das erste Material ein Kunststoff ist oder einen Kunststoff aufweist, wobei insbesondere der besagte Kunststoff einer der folgenden Kunststoffe ist: ein thermoplastisches Elastomer, Polyetherblockamid, ein thermoplastisches Polyurethan, ein Polycarbonat-basiertes thermoplastisches Polyurethan, ein Polyether-basiertes thermoplastisches Polyurethan. Hierbei kann das erste Material einen Flexular Modulus aufweisen, der im Bereich von 7 MPa bis 650 MPa liegt.

Bevorzugt ist weiterhin vorgesehen, dass das zweite Material ein Kunststoff ist oder einen Kunststoff aufweist, wobei insbesondere der besagte Kunststoff einer der folgenden Kunststoffe ist: ein thermoplastisches Elastomer, Polyetherblockamid, ein thermoplastisches Polyurethan, ein Polycarbonat-basiertes thermoplastisches Polyurethan, ein Polyether-basiertes thermoplastisches Polyurethan. Weiterhin kann hierbei das zweite Material einen anderen Flexular Modulus als das erste Material aufweisen, der im Bereich von 7 MPa bis 650 MPa liegt.

In einer Verwendungsform liegt der Bereich des ersten Materials proximal zum Operateur, also vom Patienten weg und das erste Material hat einen größeren Flexural Modulus als das zweite Material. In einer anderen Verwendungsform liegt der Bereich des ersten Materials distal zum Operateur, also zum Patienten weg und das erste Material hat einen größeren Flexural Modulus als das zweite Material. Bevorzugt ist die Variante, in der der weniger flexible Bereich proximal verwendet wird. Ferner sind auch weitere Ausführungsformen denkbar, die mehr als zwei Bereiche, zum Beispiel drei Bereiche aufweisen und Abfolgen bereithalten wie hart-weich-hart oder weich-hart-weich, wobei hier hart "weniger flexibel" und weich "flexibler" bedeuten soll.

Bevorzugt ist der erste Abschnitt 11 weiterhin in dem Verbindungsbereich V mit dem zweiten Abschnitt 12 materialschlüssig verbunden, z. B. durch ein Verschmelzen der beiden Abschnitte bzw. Materialen. Auch andere Arten der Verbindung, insbesondere stoffschlüssige Verbindungen, sind denkbar.

Hierzu können die beiden Abschnitte 11, 12 zunächst in separater Form bereitgestellt werden, wobei der jeweilige Abschnitt 11, 12 der herzustellenden Hülle 10a eine entsprechend mäanderförmige Stirnseite 11a bzw. 12a erhält, die jeweils dem Verlauf des späteren Verbindungsbereiches V folgen. Dabei weisen die beiden Stirnseiten 11a, 12a eine komplementäre Form auf, so dass sie bevorzugt formschlüssig aneinander angeordnet werden können, wobei die beiden Abschnitte 11, 12 in Richtung der Längsachse bzw. in axialer Richtung A miteinander fluchten bzw. einander gegenüberliegen.

In dieser Anordnung der beiden Abschnitte 11, 12 zueinander (siehe Figur 1) kann nun eine materialschlüssige Verbindung oder eine sonstige Verbindung zwischen den beiden Stirnseiten 11a, 12a hergestellt werden, so dass die beiden Abschnitte 11, 12 zu einer einstückigen Hülle 10a des Einführelementes 10 miteinander verbunden bzw. verschmolzen werden, wobei der besagte Verbindungsbereich V ausgebildet wird (siehe oben).

Fig. 3 zeigt schließlich zur weiteren Veranschaulichung der Erfindung eine Einführeinrichtung 1zum Positionieren und insbesondere Freisetzen eines medizinischen Implantats 20 an einem Zielort in einem menschlichen oder tierischen Körper, bei der das erfindungsgemäße Einführelement 10 Verwendung finden kann.

Die Einführvorrichtung 1 umfasst einen Innenschaft 30 und einen diesen umgebenden Außenschaft 40, an dessen proximalen Enden eine übliche, nicht naher beschriebene Handhabeeinrichtung 60 mit verschiedenen Anschlusstucken, z. B. zum Spülen der Lumen von oder zwischen Innenschaft 30 und Außenschaft 40 und zum Freigeben des Implantats 20, angeordnet ist. Zwischen den entgegengesetzt gelegenen distalen Enden ist ein Implantat 20 mit einer üblichen Führungseinrichtung 31 auf dem Innenschaft 30 angeordnet.

Zum Einführen des Katheters bzw. des Ensembles von Außenschaft 40, dem vom Außenschaft 40 umgebenen Innenschaft 30, das auf dem Innenschaft 30 angeordnete Implantat 20 mit Führungseinrichtung 31, in den menschlichen oder tierischen Körper wird dieser mit dem Implantat 20 bzw. der Führungseinrichtung 31 voran z. B. durch eine Schleuse 80 geschoben, die zuvor in das betreffende Blutgefäß eingeführt worden ist, so dass der Katheter durch die Schleuse 80 in das Blutgefäß gleiten kann.

Bei der in der Figur 3 gezeigten Einführeinrichtung 1 kann nun das erfindungsgemäße Einführelement 10 prinzipiell für alle schlauchartigen Komponenten verwendet werden.

So kann das erfindungsgemäße Einführelement 10 z. B. als Innenschaft 30 oder Außenschaft 40 verwendet werden bzw. zumindest einen Abschnitt oder einen Teil eines solchen Schaftes bilden.

Weiterhin ist auch denkbar, das Einführelement 10 als Schleuse 80 oder als Abschnitt oder Teil der Schleuse 80 zu verwenden, wie es in der Figur 3 angedeutet ist.

Weiterhin kann das Einführelement 10 im Rahmen einer Einführeinrichtung 1 auch als Stabilisator für den Außenschaft 40 verwenden werden, der den Außenschaft 50 umgibt (in der Figur 2 nicht gezeigt).

Mit Hilfe der erfindungsgemäßen Lösung kann auf einfache, kostengünstige und prozesssichere Weise die Biegesteifigkeit eines Einführelementes 10 in axialer Richtung A graduell verändert werden, so dass Steifigkeitssprünge, die üblicherweise auftreten, mit Vorteil vermieden werden können.

## Patentansprüche

1. Einführelement (10) für eine Einführeinrichtung (1), die zum Positionieren eines medizinischen Implantats (20) an einem Zielort in einem menschlichen oder tierischen Körper ausgebildet ist, wobei das Einführelement (10) eine in einer axialen Richtung (A) erstreckte Hülle (10a) aufweist, wobei die Hülle (10a) zumindest einen ersten Abschnitt (11) und einen den ersten Abschnitt (11) in der axialen Richtung (A) gegenüberliegenden zweiten Abschnitt (12) aufweist, wobei die beiden Abschnitte (11, 12) über einen in der Umfangsrichtung (U) der Hülle (10a) umlaufenden Verbindungsbereich (V) miteinander verbunden sind, und wobei der erste Abschnitt (11) aus einem ersten Material und der zweite Abschnitt (12) aus einem zweiten Material gefertigt ist, wobei das erste Material eine größere Biegesteifigkeit aufweist als das zweite Material,
**dadurch gekennzeichnet,**
**dass** der Verbindungsbereich (V) mäanderförmig in der Umfangsrichtung (U) umläuft.

2. Einführelement nach Anspruch 1, **dadurch gekennzeichnet, dass** der mäanderförmige Verlauf des Verbindungsbereiches (V) durch in der Umfangsrichtung (U) alternierend angeordnete berg- und talförmige Abschnitte (B, T) des Verbindungsbereiches (V) gebildet ist.

3. Einführelement nach Anspruch 1 oder 2, **dadurch gekennzeichnet, dass** eine Basis (B') des jeweiligen bergförmigen Abschnittes (B) kleiner ist als dessen Höhe (H).

4. Einführelement nach einem der vorhergehenden Ansprüche, **dadurch gekennzeichnet, dass** jeder bergförmige Abschnitt (B) durch je zwei Schenkel (S) gebildet ist, wobei die beiden Schenkel (S) des jeweiligen bergförmigen Abschnitts (B) einen Winkel (W) bilden, der vorzugsweise im Bereich von 5° bis 60° liegt, und/oder dass jeder bergförmige Abschnitt (B) durch je zwei Schenkel (S') gebildet ist, wobei die beiden Schenkel (S') des jeweiligen talförmigen Abschnitts (T) einen Winkel (W') bilden, der vorzugsweise im Bereich von 1° bis 60° liegt.

5. Einführelement, nach Anspruch 2 oder einem der Ansprüche 3 bis 4, sofern rückbezogen auf Anspruch 2, **dadurch gekennzeichnet, dass** die tal- und bergförmigen Abschnitte (B, T) des Verbindungsbereiches (V) jeweils rechteckförmig, trapezförmig, dreieckförmig, oder stetig gekrümmt ausgebildet sind.

6. Einführelement nach einem der vorhergehenden Ansprüche, **dadurch gekennzeichnet, dass** das erste Material ein Kunststoff ist oder einen Kunststoff aufweist, wobei insbesondere der besagte Kunststoff einer der folgenden Kunststoffe ist: Polyetherblockamid, ein thermoplastisches Polyurethan, ein thermoplastisches Elastomer, ein Polycarbonat-basiertes thermoplastisches Polyurethan, ein Polyether-basiertes thermoplastisches Polyurethan.

7. Einführelement nach einem der vorhergehenden Ansprüche, **dadurch gekennzeichnet, dass** das zweite Material ein Kunststoff ist oder einen Kunststoff aufweist, wobei insbesondere der besagte Kunststoff einer der folgenden Kunststoffe ist: Polyetherblockamid, ein thermoplastisches Polyurethan, ein thermoplastisches Elastomer, ein Polycarbonat-basiertes thermoplastisches Polyurethan, ein Polyether-basiertes thermoplastisches Polyurethan.

8. Einführelement nach einem der vorhergehenden Ansprüche, **dadurch gekennzeichnet, dass** das erste Material einen Flexular Modulus aufweist, der im Bereich von 7 MPa bis 650 MPa liegt.

9. Einführelement nach einem der vorhergehenden Ansprüche, **dadurch gekennzeichnet, dass** das zweite Material einen vom ersten Material abweichenden Flexular Modulus aufweist, der im Bereich von 7 MPa bis 650 MPa liegt.

10. Einführelement nach einem der vorhergehenden Ansprüche, **dadurch gekennzeichnet, dass** der erste Abschnitt (11) und der zweite Abschnitt (12) über den Verbindungsbereich (V) materialschlüssig miteinander verbunden sind, insbesondere durch Verschmelzen der beiden Materialien.

11. Einführeinrichtung (1), die zum Positionieren eines medizinischen Implantats (20) an einem Zielort in einem menschlichen oder tierischen Körper ausgebildet ist, **dadurch gekennzeichnet, dass** die Einführeinrichtung (1) ein Einführelement (10) nach einem der vorhergehenden Ansprüche aufweist, wobei das Einführelement (10) zumindest eine der folgenden Komponenten der Einführeinrichtung (1) bildet oder zumindest einen Teil einer der folgenden Komponenten der Einführeinrichtung (1) bildet:
- ein Innenschaft (30) zum Tragen des Implantats (20);
- ein Außenschaft (40), der einen Innenschaft (20) der Einführeinrichtung (1) umgibt, der zum Tragen des Implantats (20) ausgebildet ist;
- eine Schleuse (80), über die ein Außenschaft (40) der Einführeinrichtung (1) zusammen mit einem im Außenschaft (40) geführten Innenschaft (30) der Einführeinrichtung (1), der zum Tragen des Implantats (20) dient, in ein Körperlumen eines menschlichen oder tierischen Körpers einführbar ist;
- ein Stabilisator, der zum Stabilisieren eines Außenschafts (40) der Einführeinrichtung (1) dient und den Außenschaft (40) bevorzugt umgibt, wobei im Außenschaft (40) ein Innenschaft (30) der Einführeinrichtung (1) geführt ist, der zum Tragen des Implantats (20) dient.

12. Verfahren zur Herstellung eines Einführelementes (10) nach einem der vorgehenden Ansprüche, aufweisend die Schritte:
- Bereitstellen zumindest eines ersten und eines zweiten Abschnittes (11, 12) einer Hülle (10a) des Einführelementes (10), wobei der erste Abschnitt (11) aus einem ersten Material und der zweite Abschnitt (12) aus einem zweiten Material gebildet ist, wobei das erste Material eine größere Biegesteifigkeit als das zweite Material aufweist, wobei der erste Abschnitt (11) eine umlaufende Stirnseite (11a) mit einem mäanderförmigen Verlauf ausweist und wobei der zweite Abschnitt (12) eine Stirnseite (12a) mit einem mäanderförmigen Verlauf aufweist, der komplementär zum Verlauf der ersten Stirnseite (11a) ausgebildet ist, so dass die beiden Stirnseiten (11a, 12a) formschlüssig aneinander anliegen können,
- Anordnen der beiden Abschnitte zueinander, derart, dass die beiden Stirnseiten aneinander anliegen, insbesondere formschlüssig, und
- Ausbilden einer insbesondere materialschlüssigen Verbindung zwischen den beiden Stirnseiten (11a, 12a) so dass die beiden Abschnitte (11, 12) zu einer einstückigen Hülle (10a) des Einführelementes (10) miteinander verbunden werden.

13. Verfahren nach Anspruch 12, **dadurch gekennzeichnet, dass** die materialschlüssige Verbindung durch Verschmelzen der beiden Stirnseiten (11a, 12a) hergestellt wird.

## Claims

1. An insertion element (10) for an insertion device (1), which is designed to position a medical implant (20) in a target location in a human body or an animal body, the insertion element (10) comprising a sleeve (10a) that extends in an axial direction (A), the sleeve (10a) comprising at least one first section (11) and a second section (12) located opposite the first section (11) in the axial direction (A), the two sections (11, 12) being connected to one another by a connection region (V) extending circumferentially in the circumferential direction (U) of the sleeve (10a), the first section (11) being made of a first material and the second section (12) being made of a second material, the first material having greater flexural rigidity than the second material,
**characterized in**
**that** the connection region (V) extends circumferentially around in the circumferential direction (U) in a meander-shaped manner.

2. The insertion element according to claim 1, **characterized in that** the meander-shaped course of the connection region (V) is formed by peak- and valley-shaped sections (B, T) of the connection region (V) which are arranged alternately in the circumferential direction (U).

3. The insertion element according to claim 1 or 2, **characterized in that** a base (B') of the respective peak-shaped section (B) is smaller than the height (H) thereof.

4. The insertion element according to any one of the preceding claims, **characterized in that** each peak-shaped section (B) is formed by two legs (S), the two legs (S) of the respective peak-shaped section (B) forming an angle (W) that is preferably in the range of 5° to 60°, and/or that each peak-shaped section (B) is formed by two legs (S'), the two legs (S') of the respective valley-shaped section (T) forming an angle (W') that is preferably in the range of 1° to 60°.

5. The insertion element according to claim 2 or any one of claims 3 to 4, provided these have a back-reference to claim 2, **characterized in that** the valley- and peak-shaped sections (B, T) of the connection region (V) each have a rectangular, trapezoidal, triangular or steadily curved design.

6. The insertion element according to any one of the preceding claims, **characterized in that** the first material is a plastic material or comprises a plastic material, in particular said plastic material being one of the following plastic materials: polyether block amide, a thermoplastic polyurethane, a thermoplastic elastomer, a polycarbonate-based thermoplastic polyurethane, and a polyether-based thermoplastic polyurethane.

7. The insertion element according to any one of the preceding claims, **characterized in that** the second material is a plastic material or comprises a plastic material, in particular said plastic material being one of the following plastic materials: polyether block amide, a thermoplastic polyurethane, a thermoplastic elastomer, a polycarbonate-based thermoplastic polyurethane, and a polyether-based thermoplastic polyurethane.

8. The insertion element according to any one of the preceding claims, **characterized in that** the first material has a flexural modulus that is in the range of 7 MPa to 650 MPa.

9. The insertion element according to any one of the preceding claims, **characterized in that** the second material has a flexural modulus, deviating from the flexural modulus of the first material, that is in the range of 7 MPa to 650 MPa.

10. The insertion element according to any one of the preceding claims, **characterized in that** the first section (11) and the second section (12) are integrally bonded to one another via the connection region (V), in particular by fusion of the two materials.

11. An insertion device (1), which is designed to position a medical implant (20) in a target location in a human body or an animal body, **characterized in that** the insertion device (1) comprises an insertion element (10) according to any one of the preceding claims, the insertion element (10) forming at least one of the following components of the insertion device (1) or forming at least part of one of following components of the insertion device (1):
- an inner shaft (30) to carry the implant (20);
- an outer shaft (40) surrounding an inner shaft (20) of the insertion device (1) designed to carry the implant (20);
- a sheath (80) by way of which an outer shaft (40) of the insertion device (1) can be inserted, together with an inner shaft (30) of the insertion device (1) which is guided in the outer shaft (40) and used to carry the implant (20), into a body lumen of a human body or an animal body; and
- a stabilizer, which is used to stabilize an outer shaft (40) of the insertion device (1) and preferably surrounds the outer shaft (40), an inner shaft (30) of the insertion device (1) that is used to carry the implant (20) being guided in the outer shaft (40).

12. A method for producing an insertion element (10) according to any one of the preceding claims, comprising the following steps:
- providing at least one first and one second section (11, 12) of a sleeve (10a) of the insertion element (10), the first section (11) being made of a first material and the second section (12) being made of a second material, the first material having greater flexural rigidity than the second material, the first section (11) including a circumferential end face (11a) having a meander-shaped course, and the second section (12) including an end face (12a) having a meander-shaped course that is complementary to the course of the first end face (11a) so that the two end faces (11a, 12a) can rest against one another in a form-locked manner;
- arranging the two sections with respect to one another in such a way that the two end faces rest against one another, in particular in a form-locked manner; and
- forming an in particular integrally bonded connection between the two end faces (11a, 12a) so that the two sections (11, 12) are connected to one another to form a single-piece sleeve (10a) of the insertion element (10).

13. The method according to claim 12, **characterized in that** the integrally bonded connection is established by fusing the two end faces (11a, 12a).

## Revendications

1. Elément d'insertion (10) pour un dispositif d'insertion (1), qui est conçu pour le positionnement d'un implant (20) médical à un endroit cible dans un corps humain ou animal, où l'élément d'insertion (10) présente une gaine (10a) allongée dans une direction axiale (A), où la gaine (10a) présente au moins un premier segment (11) et un deuxième segment (12), situé en face du premier segment (11), dans la direction axiale (A), où les deux segments (11, 12) sont reliés l'un à l'autre par le biais d'une zone de liaison (V) entourant la gaine (10a) dans la direction circonférentielle (U), et où le premier segment (11) est fabriqué à base d'un premier matériau et le deuxième segment (12) à base d'un deuxième matériau, où le premier matériau présente une rigidité en flexion supérieure au deuxième matériau,
**caractérisé en**
**ce que** la zone de liaison (V) s'étend en forme de méandres dans la direction circonférentielle (U).

2. Elément d'insertion selon la revendication 1, **caractérisé en ce que** l'évolution en forme de méandres de la zone de liaison (V) est constituée par des segments en forme de sommets et de creux (B, T) de la zone de liaison (V) disposés alternativement dans la direction circonférentielle (U).

3. Elément d'insertion selon la revendication 1 ou la revendication 2, **caractérisé en ce qu'**une base (B') du segment en forme de sommet (B) respectif est plus petite que sa hauteur (H).

4. Elément d'insertion selon l'une des revendications précédentes, **caractérisé en ce que** chaque segment en forme de sommet (B) est formé par deux épaulements (S), où les deux épaulements (S) du segment en forme de sommet (B) respectif forment un angle (W) qui se situe de préférence dans la plage de 5 ° à 60 °, et/ou que chaque segment en forme de sommet (B) est formé par deux épaulements (S'), où les deux épaulements (S) du segment en forme de creux (T) respectif forment un angle (W') qui se situe de préférence dans la plage de 1 ° à 60 °.

5. Elément d'insertion selon la revendication 2 ou une des revendications 3 à 4, dans la mesure où elles sont en relation avec la revendication 2, **caractérisé en ce que** les segments en forme de creux et de sommets (B, T) de la zone de liaison (V) sont conçus respectivement en forme de rectangles, en forme de trapèzes, en forme de triangles, ou sont régulièrement incurvés.

6. Elément d'insertion selon l'une des revendications précédentes, **caractérisé en ce que** le premier matériau est une matière plastique ou présente une matière plastique, où, de préférence, ladite matière plastique est l'une des matières plastiques suivantes : du polyéther bloc amide, un polyuréthane thermoplastique, un élastomère thermoplastique, un polyuréthane thermoplastique basé sur du polycarbonate, un polyuréthane thermoplastique basé sur du polyéther.

7. Elément d'insertion selon l'une des revendications précédentes, **caractérisé en ce que** le deuxième matériau est une matière plastique ou présente une matière plastique, où, de préférence, ladite matière plastique est l'une des matières plastiques suivantes : du polyéther bloc amide, un polyuréthane thermoplastique, un élastomère thermoplastique, un polyuréthane thermoplastique basé sur du polycarbonate, un polyuréthane thermoplastique basé sur du polyéther.

8. Elément d'insertion selon l'une des revendications précédentes, **caractérisé en ce que** le premier matériau présente un module de flexion qui se situe dans la plage de 7 MPa à 650 MPa.

9. Elément d'insertion selon l'une des revendications précédentes, **caractérisé en ce que** le deuxième matériau présente un module de flexion différent du premier matériau, qui se situe dans la plage de 7 MPa à 650 MPa.

10. Elément d'insertion selon l'une des revendications précédentes, **caractérisé en ce que** le premier segment (11) et le deuxième segment (12) sont reliés l'un à l'autre par complémentarité des matières par le biais de la zone de liaison (V), en particulier par une fusion des deux matériaux.

11. Dispositif d'insertion (1) qui est conçu pour le positionnement d'un implant (20) médical à un endroit cible dans un corps humain ou animal, **caractérisé en ce que** le dispositif d'insertion (1) présente un élément d'insertion (10) selon l'une des revendications précédentes, où l'élément d'insertion (10) forme au moins un des composants du dispositif d'insertion (1) ou forme au moins une partie des composants suivants du dispositif d'insertion (1) :
- une tige intérieure (30) pour supporter l'implant (20) ;
- une tige extérieure (40), qui entoure une tige intérieure (20) du dispositif d'insertion (1), qui est conçue pour supporter l'implant (20) ;
- un sas (80), par le biais duquel une tige extérieure (40) du dispositif d'insertion (1) conjointement avec une tige intérieure (30) du dispositif d'insertion (1) menée dans la tige extérieure, qui sert à supporter l'implant (20), peut être inséré dans une lumière corporelle d'un corps humain ou animal ;
- un stabilisateur qui sert à stabiliser une tige extérieure (40) du dispositif d'insertion (1) et qui de préférence entoure la tige extérieure (40), où une tige intérieure (30) du dispositif d'insertion (1) est menée dans la tige extérieure (40), qui sert à supporter l'implant (20).

12. Procédé de fabrication d'un élément d'insertion (10) selon l'une des revendications précédentes, présentant les étapes :
- de fourniture d'au moins un premier et d'un deuxième segment (11, 12) d'une gaine (10a) de l'élément d'insertion (10), où le premier segment (11) est fabriqué à base d'un premier matériau et le deuxième segment (12) à base d'un deuxième matériau, où le premier matériau présente une rigidité en flexion supérieure au deuxième matériau, où le premier segment (11) présente une face frontale (11a) périphérique avec une évolution en forme de méandre et où le deuxième segment (12) présente une face frontale (12a) périphérique avec une évolution en forme de méandre, qui est complémentaire à l'évolution de la première face frontale (11a), de sorte que les deux faces frontales (11a, 12a) peuvent se plaquer l'une à l'autre par complémentarité des formes,
- d'agencement des deux segments l'un par rapport à l'autre de sorte que les deux faces frontales se plaquent l'une à l'autre, notamment par complémentarité des formes, et
- de formation d'une liaison, notamment par complémentarité des matières, entre les deux faces frontales (11a, 12a) de sorte que les deux segments (11, 12) sont reliés l'un à l'autre en formant une gaine (10a) en une pièce de l'élément d'insertion '10).

13. Procédé selon la revendication 12, **caractérisé en ce que** la liaison par complémentarité des matières est faite par la fusion des deux faces frontales (11a, 12a).
